# EUROPEAN PATENT APPLICATION

(11) **EP 2 597 157 A1**
(43) Date of publication of application: **29.05.2013**
(21) Application number: 12193878.1
(22) Date of filing: 22.11.2012
(51) Int. Cl.: C12P 19/18, C07H 3/06, A23L 1/09

(54) **High purity gentiooligosaccharides obtained therefrom and uses thereof**

(30) Priority: 25.11.2011 KR 20110012445
(71) Applicant: Corn Products Development, Inc., Bridgewater, NJ 08807 (US)
(72) Inventor: Lee, Jae Ho, 404-851 Incheon (KR); Ahn, Sang Wook, 200-780 Gangwon-do (KR); Park, Sang Jae, 448-517 Gyeonggi-do (KR); Kim, Kyuong Hee, 200-781 Gangwon-do (KR)
(74) Representative: Held, Stephan

(57) **Abstract**

The present invention relates to methods for preparing high purity gentiooligosaccharides, high purity gentiooligosaccharides obtained therefrom, and uses thereof. The methods of the present invention involve: adding a low purity gentiooligosaccharide to a liquid medium; subjecting the liquid medium to inoculation with a microorganism, followed by incubation and fermentation to consume glucose that is contained in the low purity gentiooligosaccharide; and subjecting the resulting fermentation broth to filtration and purification. According to the method of the present invention, high purity gentiooligosaccharides having a purity of at least 90% that can be used as alternatives to foods such as cocoa, chocolate, coffee, beer, tea, bread or confectionery product, and beverage or the main ingredients thereof.

## Description

### FIELD OF THE INVENTION

The present invention relates to methods for preparing high purity gentiooligosaccharides, high purity gentiooligosaccharides obtained therefrom and uses thereof, and more particularly to methods for preparing high purity gentiooligosaccharides that can be beneficially used as alternatives to foods such as cocoa, chocolate, coffee, beer, tea, bread or confectionery products, beverages, etc., and main ingredients thereof without producing unnecessary by-products by fermenting low purity gentiooligosaccharides containing large amounts of glucose with microorganisms to remove glucose therefrom.

### BACKGROUND OF THE INVENTION

Gentiooligosaccharides are sweeteners composed of mainly gentiobiose and cellobiose that are disaccharides synthesized by enzymes from glucose, and are the generic name of oligosaccharides having ·-1,6-glucoside bonds. Gentiooligosaccharides are composed of gentiobiose, cellobiose, gentiotriose, gentiotetraose, etc., and their main ingredient is gentiobiose which is a disaccharide having a strong bitter taste.

Gentiooligosaccharides are found in the roots or stems of plants and in natural honey and are prepared by an enzymatic reaction using starch as raw material. Gentiooligosaccharides are known as low digestible sugars that aid in mineral absorption, are selectively utilized by Bifidobacterium and Lactobacillus to help improve intestinal microbial flora, and have an intestinal regulation effect similar to that of galactooligosaccharide which has an excellent intestinal regulation effect.

Gentiooligosaccharides are characterized by having a softer and fresher bitterness in comparison with substances having a bitter taste, such as Naringin. The bitter taste of gentiooligosaccharides, in harmony with the bitter taste of various foods such as chocolate, cocoa, coffee, beer, tea, etc.., lessens the astringency of fruits and vegetables and softens the bitterness of foods having a bitter taste and increases preferences to food by the addition of small amounts. Gentiooligosaccharides can be applied to various foods, for example, fruit juices such as orange juice and pineapple juice, coffee, vegetable drinks, pumpkin congee, *etc.*

Such gentiooligosaccharides can be prepared industrially from a high concentration of glucose by condensation and transglucosylation reactions using a fungal ·-glucosidase (see [Umino Takehiro: Study of Industrial Production of Gentiooligosaccharide, Journal of Applied Glycoscience (Japan), 42, 83 (1995)]). Currently, gentiooligosaccharides having a purity as high as 45% are produced by enzymatic synthesis, and those having a purity as high as 90% or higher are produced by using cation exchange resins. Here, the remaining components other than gentiooligosaccharides are primarily glucose, and for example, gentiooligosaccharides having a purity of 45% contain about 50 to 55% by weight of glucose, based on the total weight of saccharide components, while gentiooligosaccharides having a purity of 90% contain about 0.5 to 5% by weight of glucose based on the total weight of saccharide components.

Glucose has about 60% of the sweetness of sucrose. Sometimes, glucose contained in gentiooligosaccharides plays a role in providing sweetness, but there are instances where it inhibits the manifestation of bitterness depending on the subjects to which it is applied. In addition, it is difficult to apply gentiooligosaccharides to subjects due to the glucose contained therein. For example, in the case of chocolates, the addition of gentiooligosaccharides containing a high content of glucose may cause difficulties in molding. Accordingly, there may be a need to use gentiooligosaccharides having high purities depending on the subjects to which they are applied. Further, the higher the purity of oligosaccharides, one can obtain the effect with less amount, thereby making it possible to achieve the same effects with small amounts. Thus, the preparation of high purity gentiooligosaccharides is essential.

However, practically there are a number of limitations in preparing high purity gentiooligosaccharides. Previously, a case has been reported where the purity of gentiooligosaccharides was raised to about 90% or higher by columns utilizing ion exchange resins (see [Umino Takehiro: Study of Industrial Production of Gentiooligosaccharide, Journal of Applied Glycoscience (Japan), 42, 85-91 (1995)]). However, according to column chromatography, the purity is determined depending on the degree of separation during the preparation of high purity gentiooligosaccharides, but there are disadvantages in that when separating gentiooligosaccharides with high purity, the amount of the by-product fractions increases and gentiooligosaccharides are eluted with the glucose fractions, resulting in a low yield. Furthermore, in cases where by-product fractions are sought to be used for other purposes, the by-product fractions need to be subject to retreatment such as decomposition of gentiooligosaccharides into glucose by low concentrations of enzyme, since large amounts of glucose and small amounts of gentiooligosaccharide are contained therein.

### DISCLOSURE OF THE INVENTION

### PROBLEMS TO BE SOLVED BY THE INVENTION

It is an object of the present invention to provide methods for preparing high purity gentiooligosaccharides having a purity of at least 90% without the production of unnecessary by-products.

It is another object of the present invention to provide high purity gentiooligosaccharides having a purity of at least 90% prepared by the above methods.

It is yet another object of the present invention to provide methods of improving food taste by adding the above high purity gentiooligosaccharides having a purity of at least 90% to foods.

It is still another object of the present invention to provide methods of using the above high purity gentiooligosaccharides having a purity of at least 90% as alternatives to foods or food additives.

### MEANS TO SOLVE THE PROBLEMS

In order to achieve the above objectives, there are provided methods for preparing high purity gentiooligosaccharides comprising: adding a low purity gentiooligosaccharide to a liquid medium; subjecting the liquid medium to inoculation with a microorganism, followed by incubation and fermentation to consume glucose that is contained in the low purity gentiooligosaccharide; removing the microorganism from the obtained fermentation broth e.g., by filtration and centrifugation; and subjecting the obtained broth to purification.

Also, the present invention provides high purity gentiooligosaccharides having a purity of at least 90% prepared by the above methods.

In addition, the present invention provides methods of improving food taste by adding the above high purity gentiooligosaccharides having a purity of at least 90% to foods.

Further, the present invention provides methods of using the above high purity gentiooligosaccharides having a purity of at least 90% as alternatives to foods or food additives.

### EFFECT OF THE INVENTION

According to the methods of the present invention where low purity gentiooligosaccharides containing large amounts of glucose are subjected to inoculation with microorganisms that consume glucose as carbon sources but do not consume gentiooligosaccharides and fermentation, and then the obtained fermentation broth is subjected to filtration and purification, high purity gentiooligosaccharides having a purity of at least 90% can be prepared without producing unnecessary by-products. Also, the high purity gentiooligosaccharides having a purity of at least 90% obtained according to the present invention can improve the flavor or taste of foods such as cocoa, chocolate, coffee, beer, tea, bread or confectionery products, beverages, etc.., by the addition of small amounts and can be beneficially used as alternatives to foods or their main ingredients.

### DETAILED DESCRIPTION FOR PRACTICING THE INVENTION

The methods for preparing high purity gentiooligosaccharides according to the present invention comprise adding a low purity gentiooligosaccharide to a liquid medium; subjecting the liquid medium to inoculation with a microorganism, followed by incubation and fermentation to consume glucose that is contained in the low purity gentiooligosaccharide; removing the microorganism from the obtained fermentation broth; and subjecting the broth to purification.

The low purity gentiooligosaccharides that are used in the present invention as raw material are not particularly limited, and any gentiooligosaccharides that are obtained by the conventional methods of preparing gentiooligosaccharide, e.g., by condensation and transglucosylation reactions using a fungal beta-glucosidase from a high concentration of glucose (see [Umino Takehiro: Study of Industrial Production of Gentiooligosaccharide, Journal of Applied Glycoscience (Japan), 42, 83 (1995)]), or gentiooligosaccharides obtained by conventional gentiooligosaccharide preparation processes and then initially purified with columns utilizing ion exchange resins to enhance purity may be used. The low purity gentiooligosaccharides that are used in the present invention contain about 15 to 65% by weight of glucose and can be added in solid concentrations of 5 to 70% (w/v), based on the volume of the culture medium.

In addition, the culture medium for fermentation of the present invention may contain 0.1 to 1.5% (w/v) of yeast extract, 0.1 to 0.5% (w/v) of malt extract, and 0.1 to 0.8% (w/v) of peptone as culture medium components.

Among the above culture medium components, those such as yeast extract, malt extract, and peptone are added for the growth of microorganisms for removing the glucose contained in the gentiooligosaccharides, and it is desirable that such components are added in amounts within the above ranges for the efficient culture of microorganisms.

The yeast extracts included in the liquid medium for the fermentation of the present invention are water-soluble exudates that are extracted from yeasts and are well known in the art. They are superior stimuli for the growth of microorganisms, and play a role in supplying the incubation medium with vitamins, nitrogen, amino acids, carbon, etc. It is desirable that the yeast extracts are used in the concentration of 0.1 to 1.5% (w/v) based on the culture medium.

The malt extracts are obtained by growing barley grain with yeast and mold. Further, since they contain high concentrations of carbohydrates, maltose in particular, malt extracts are suitable for the culture of microorganisms such as yeast and fungi and play a role in supplying the medium with nutrients such as carbon and proteins. It is desirable that the malt extracts are used in the concentration of 0.1 to 0.5% (w/v) based on the culture medium.

Peptones are yellow powders of small molecules containing trace amounts of proteose, which are obtained by the digestion of proteins such as animal meat and casein using enzymes such as pepsin, trypsin, pancreatine, and are used as nitrogen sources in the culture medium. It is desirable that peptones are used in the concentration of 0.1 to 0.8% (w/v) based on the culture medium.

In addition, the liquid medium for the fermentation of the present invention may contain small amounts of additional components, such as an antifoaming agent, for the efficient culture when foam is generated during microorganism fermentation for the consumption of glucose contained in the gentiooligosaccharides.

As the microorganisms used in the fermentation for obtaining high purity gentiooligosaccharides according to the present invention, those that consume glucose as carbon sources while not consuming gentiooligosaccharides, for example, fermentation fungi such as yeast, bacteria and mold may be used. Representative examples of microorganisms include yeast strains, for example, those belonging to Saccharomyces sp. such as *Saccharomyces cerevisiae, Saccharomyces carlsbergensis, Saccharomyces uvarum, Saccharomyces ellipsoideus, Saccharomyces bayanus.* Besides these, those belonging to Bacillus sp., Lactobacillus sp., and Candida sp. may be used. In particular, yeasts are desirable in that they can be easily separated and removed from the liquid medium after the fermentation is terminated, and the removed yeast may be sold as by-product goods such as animal feed.

In the present invention, it is desirable that microorganisms that are used for the fermentation for removing glucose contained in low purity gentiooligosaccharides are added at a concentration of about 10% (v/v) relative to the amount of the culture medium. Adding microorganisms in amounts exceeding the above amount is problematic in that efficiency is lowered due to over fermentation, whereas using microorganisms in amounts less than the above amount have problems in that the incubation time is prolonged.

In the present invention, fermentation may be carried out under conditions of a temperature ranging from 25 °C to 35 °C, an aeration ranging from 0.5 to 2 v/v/m (air volume/working volume/minute) and a stirring rate of 200 rpm to 500 rpm for 24 hours to 72 hours.

As described above, if a culture medium containing low purity gentiooligosaccharides is subjected to inoculation with microorganisms, incubation and fermentation, glucose that is contained in the low purity gentiooligosaccharides are consumed by microorganisms and removed, thereby making it possible to obtain high purity gentiooligosaccharides.

After the fermentation is terminated, the microorganisms that are used in the fermentation may be removed from the fermentation broth e.g., by using centrifugation or filtration, and then the obtained broth may be subjected to a purification process including decolorization using active carbon and demineralization using ion exchange resins and evaporated under reduced pressure, thereby making it possible to obtain high purity gentiooligosaccharides having a purity of at least 90%.

The high purity gentiooligosaccharides having a purity of at least 90% obtained according to the methods of the present invention have saccharide compositions as shown in Table 1 below (based on the total weight of the residual saccharides).

**<Table 1>**

| Component | Content (wt. %) |
|---|---|
| Monosaccharides including glucose | 2.0 - 5.0 |
| Disaccharides including gentiobios | 60.0 - 70.0 |
| Trisaccharides including gentiotriose | 10.0 - 20.0 |
| Tetrasaccharides and above including gentiotetraose | 5.0 -18.0 |

As such, according to the methods for preparing high purity gentiooligosaccharides by fermentation of the present invention, the glucose content in gentiooligosaccharides may be reduced to 0.5% by weight and gentiooligosaccharides having a purity of at least 90% may be easily obtained without the production of unnecessary by-products.

In addition, during the preparation of high purity gentiooligosaccharides by fermentation according to the present invention, glucose is metabolized by microorganisms, such as yeast and yeast meal, to produce ethanol. Among the byproducts of such fermentation process, yeast meal may be used as animal feed, while ethanol is expected to be in the spotlight as bio-ethanol, a new future fuel.

Further, high purity gentiooligosaccharides having a purity of at least 90% obtained according to the present invention can solve problems associated with conventional gentiooligosaccharides, where their use was limited due to the difficulties in molding or shaping of the products that are caused by the physical properties of glucose and the detrimental effects of the high glucose content thereof on sweetness. Also, they may improve the flavor and taste of various foods, such as chocolate, cocoa, beer, tea, coffee and red ginseng by the addition of small amounts. Further, they can be beneficially used as alternatives to foods such as cocoa, coffee, chocolate, tea, bread or confectionery products, and their main ingredients.

For example, in preparing bread products of cocoa, chocolate, green tea or the like, substituting high purity gentiooligosaccharides having a purity of at least 90% obtained according to the present invention for 0.1 to 40% by weight of the main ingredients of the bread products, i.e., cocoa (e.g., Sugarless 100% Cocoa Powder (Valrhona), HERSHEY'S COCOA (HERSHEY), etc..), dark chocolate (e.g., Real Choco Chip Dark (Belcolade), Le Noir 68% Baking Bar (Valrhona), *etc*..), or green tea powder, etc. may exhibit a bitterness and taste similar to those of conventional products to which no gentiooligosaccharide is added.

In addition, in the case of brownies using cocoa and dark chocolate, substituting high purity gentiooligosaccharides having a purity of at least 90% obtained according to the present invention for 0.1 to 40% by weight of dark chocolate and 0.1 to 40% by weight of cocoa, which are the main ingredients of brownies may also exhibit a bitterness and taste similar to those of conventional products to which no gentiooligosaccharide is added.

For red ginseng beverages, the proportion of high purity gentiooligosaccharides to be used varies depending on the concentration of the red ginseng concentrate to be added therein. In the case of red ginseng beverages that contain 1.5 to 2% by weight of red ginseng concentrate, substituting high purity gentiooligosaccharides having a purity of at least 90% for 0.1 to 40% by weight of red ginseng concentrate can exhibit a bitterness similar to that of the conventional beverage; whereas in the case of red ginseng beverages that contain 0.4 to 0.5% by weight of red ginseng concentrate,
substituting high purity gentiooligosaccharides having a purity of at least 90% for 0.1 to 30% by weight of red ginseng concentrate can exhibit a bitterness and taste similar to those of the conventional beverages to which no gentiooligosaccharide is added. In addition, when high purity gentiooligosaccharides having a purity of at least 90% are used instead of red ginseng concentrates as described above, high purity gentiooligosaccharides may be added by one- to ten-fold the amount of red ginseng concentrate to be substituted.

For coffee beverages, although the substituted amounts vary depending on the characteristics of the coffee beans, such as the place of origin for the coffee plant, high purity gentiooligosaccharides of the present invention may be substituted for 0.1 to 10% by weight of the coffee beans.

The present invention is further described and illustrated according to the examples provided below. However, it should be noted that the following examples are presented only for illustrative purposes and are not intended to limit the scope of the present invention.

### EXAMPLE 1:

### (1) Preparation of seed culture

30 mL of YM broth (Difco) was inoculated with 0.5 mL of the *Saccharomyces cerevisiae* Y1135 strain, followed by incubation at 30 °C for 24 hours while stirring at 180 rpm.

### (2) Incubation

The crude gentiooligosaccharide (purity 45%) was added to 50 mL of medium containing 0.3% (w/v) of yeast extract (Difco), 0.3% (w/v) of malt extract (Difco), 0.5% (w/v) of peptone (Difco), and water at the solid concentrations of 5% (w/v) and 10% (w/v), respectively, based on the volume of the medium, and was inoculated with 1 mL of the *Saccharomyces cerevisiae* Y1135 seed culture obtained in step (1), and followed by incubation for 48 hours under conditions of a culture temperature of 30oC, aeration of 1.0 v/v/m and stirring rate of 300 rpm in an incubator.

The culture broth was analyzed using high performance liquid chromatography (HPLC) (Waters, HPX-42A column) and BIO-LC (DIONEX, ICS-5000 Bio-LC Chromatography System, PA detector, PA-1 column). As a result, in the case of performing incubation with the addition of crude gentiooligosaccharide (purity 45%) at the solid concentration of 5% (w/v) based on the volume of the liquid medium, gentiooligosaccharides having a gentiooligosaccharide content of 92.8% by weight based on the residual saccharides were obtained after fermentation, while in the case of performing incubation with the addition of crude gentiooligosaccharide (purity 45%) at the solid concentration of 10% (w/v), gentiooligosaccharides having a gentiooligosaccharide content of 92.0% by weight based on the residual saccharides were obtained after fermentation.

### EXAMPLE 2:

The crude gentiooligosaccharide (purity 45%) was added to 3 L of medium containing 0.3% (w/v) of yeast extract (Difco), 0.3% (w/v) of malt extract (Difco), 0.5% (w/v) of peptone (Difco), 1 mL of anti-foaming agent (Dow Corning, LS-300), and water at the solid concentrations of 30% (w/v), 40% (w/v) and 50% (w/v), respectively, based on the volume of the medium, and was inoculated with 300 mL of the *Saccharomyces cerevisiae* Y1135 seed culture obtained in step (1), followed by incubation for 48 hours under conditions of a culture temperature of 30oC, aeration of 1.0 v/v/m and stirring rate of 300 rpm in a 5 L incubator. The gentiooligosaccharide containing culture broth obtained after incubation comprises, besides gentiooligosaccharides and glucose, glycerol and monosaccharide ingredients (e.g., glucose, fructose, *etc.*) resulting from the liquefaction and glycosylation of starch. The culture broth was subject to filtration to remove the microorganisms, followed by decolorization with activated carbon and demineralization with ion exchange resins, and subsequently concentrated to obtain gentiooligosaccharide powder by using a spray dryer. The contents of the ingredients in the powder (based on the total weight of residual saccharides) were analyzed by a similar manner as in Example 1. The results are shown in Table 2 below.

**<Table 2>**

| Amount of crude gentiooligosaccharide added [% (w/v)] | Incubation time (hrs) | Amount of gentiooligosaccharide contained in the powder (wt. %) | Amount of monosaccharide including glucose in the powder (wt. %) |
|---|---|---|---|
| 30 | 48 | 94.5 | 5.5 |
| 40 | 48 | 95.2 | 4.8 |
| 50 | 48 | 92.4 | 7.6 |

As shown in Table 2, high purity gentiooligosaccharides having a purity of 90% or higher can be obtained by fermentation regardless of what concentration of crude gentiooligosaccharides is added.

### EXAMPLE 3:

In preparing brownies having dark chocolate (Real Chocochip Dark from Belcolade (Belgium), ingredients: cocoa weight 45.8%, cocoa butter 9.9%, sugar, lecithin, natural vanilla flavor, etc..), and cocoa powder (Sugarless 100% Cocoa Powder from Valrhona (France)) as the main ingredients, Experimental Group 1 where 12 g of high purity gentiooligosaccharide (purity 95.2%) obtained in Example 2 was substituted for 7.7% by weight of the dark chocolate and 33.3% by weight of the cocoa powder; Experimental Group 2 where 10 g of high purity gentiooligosaccharide (purity 95.2%) was substituted for 6.1% by weight of the dark chocolate and 33.3% by weight of the cocoa powder; or Control Group that is made up according to conventional combination ratios were employed to prepare brownies. The respective combination ratios of each sample from Experimental Groups 1 and 2 and Control Group were shown in Table 3 below.

**<TABLE 3>**

| | Control group | Experimental group 1 | Experimental group 2 |
|---|---|---|---|
| Dark chocolate (g) | 130 | 120 | 122 |
| White sugar (g) | 76 | 76 | 76 |
| Cocoa powder (g) | 6 | 4 | 4 |
| High purity gentiooligosaccharide (g) | 0 | 12 | 10 |
| Egg (number) | 2 | 2 | 2 |
| Butter (g) | 80 | 80 | 80 |
| Flour (*1) (g) | 94 | 94 | 94 |
| Brown sugar (g) | 34 | 34 | 34 |
| Powdered skim milk (g) | 6 | 6 | 6 |
| Salt (g) | 2 | 2 | 2 |
| Vanilla extract (g) | 2 | 2 | 2 |

| | | | |
|---|---|---|---|
| (*1) Protein content: 8% | | | |

The bitterness, softness and preference for the prepared control group and experimental groups 1 and 2 were assessed and evaluated through a senso ry test.

In the sensory test, a 9-scale test where the attributes (bitterness and softness) and preference were evaluated using classification grades ranging from 1 to 9 was used. In the case of bitterness, the closer the score is to 9, it is more bitter, while in the case of softness, the closer the score is to 9, it was softer. With respect to taste preference, the closer the score is to 9, the better is the preference. The selected panelists were 6 females and 6 males of ages ranging from 25 to 40 and were educated and trained as to the sensory test for two months. The average values of bitterness, softness and preference for brownies made from the Control Group and Experimental Groups 1 and 2 are shown in Table 4 below. The Sigmaplot 11.0 program from Systat Software as used for the statistical data regarding the sensory test results, where statistically significant differences among the above three groups were examined with respect to the average values by the one-way layout design ANOVA at a significance level of 0.05, and the differences between the groups were statistically analyzed using the Tukey Test.

**<TABLE 4>**

| | Bitterness | Softness | Preference |
|---|---|---|---|
| Control group | 4.667 | 5.75 | 5.583 |
| Experimental group 1 | 5.917 | 5.917 | 5.5 |
| Experimental group 2 | 4.75 | 6.333 | 6.583 |

As a result of the sensory test on bitterness, softness and preference, the brownies of Experimental Group 1 where the high purity gentiooligosaccharide was substituted for 7.7% by weight of the dark chocolate and 33.3% by weight of the cocoa powder showed a statistically significant higher score for bitterness, but showed no statistical differences for softness and preference, in comparison to the brownies of the Control Group that contain no high purity gentiooligosaccharide. Further, the brownies of Experimental Group 2 where the high purity gentiooligosaccharide was included in lesser amounts, i.e., substituted for 6.1% by weight of the dark chocolate and the 33.3% by weight of the cocoa powder, showed no statistical significant differences for bitterness and softness, while exhibiting a statistically significant higher preference, when compared to the brownies of the Control Group.

That is to say, it can be confirmed from the results of Experimental Groups 1 and 2 that substituting high purity gentiooligosaccharides having the purity of 90% or higher of the present invention for not more than 40% by weight of the main ingredients of brownies, i.e., cocoa powder and dark chocolate, showed no sensory differences, while exhibiting improved overall taste and preference, in comparison to the conventional brownies that contain no gentiooligosaccharide.

### EXAMPLE 4:

In preparing red ginseng beverages containing 2% by weight (6 g) of red ginseng concentrate, the high purity gentiooligosaccharide (purity 92.5%) obtained in Example 2 was substituted for 25% by weight (1.5 g) of the red ginseng concentrate, while the high purity gentiooligosaccharide was added in an amount three-fold (4.5 g) the amount of the red ginseng concentrate to be substituted (1.5 g). The main ingredients and combination ratios of the red ginseng beverages are shown in Table 5 below.

**<Table 5>**

| Ingredients | Control group | Experimental group |
|---|---|---|
| Red ginseng concentrate (g) | 6 | 4.5 |
| High purity gentiooligosaccharide (g) | 0 | 4.5 |
| Glucose powder (g) | 9 | 9 |
| Crystalline fructose (g) | 25 | 25 |
| Citric acid (g) | 0.26 | 0.26 |
| Purified water (g) | 260 | 257 |
| Total weight (g) | 300 | 300 |

The red ginseng beverages of the Control Group and Experimental Group were evaluated with respect to bitterness, softness, and preference by a sensory test and the results are shown in Table 6 below. The sensory test and the statistical analysis were conducted in the same manner as described in Example 3.

**<Table 6>**

| | Bitterness | Softness | Preference |
|---|---|---|---|
| Control group | 5.833 | 3.167 | 4.583 |
| Experimental group | 5.917 | 3.083 | 4.833 |

As a result, the red ginseng beverages of the Control Group and Experimental Group exhibited no statistically significant differences at the significance level of 0.05, and showed no large differences in the total average values for bitterness, softness and preference. Accordingly, substituting high purity gentiooligosaccharide of the present invention for a portion of red ginseng concentrate that is the main ingredient of red ginseng beverages resulted in no sensory differences in bitterness, softness and preference, in comparison with the original beverages that contain no gentiooligosaccharide.

## Claims

1. A method for preparing a high purity gentiooligosaccharide comprising:
adding a low purity gentiooligosaccharide to a liquid medium;
subjecting the liquid medium to inoculation with a microorganism, followed by incubation and fermentation to consume glucose that is contained in the low purity gentiooligosaccharide;
removing the microorganism from the obtained fermentation broth; and subjecting the obtained broth to purification.

2. The method of claim 1, wherein the low purity gentiooligosaccharide contains 15 to 65% by weight of glucose and 35 to 85% by weight of gentiooligosaccharide.

3. The method of claim 1 or of claim 2, wherein the low purity gentiooligosaccharide is added to the liquid medium at a concentration of 5 to 70% (w/v).

4. The method of any one of claims 1 to 3, wherein the microorganism consumes glucose as a carbon source while not consuming gentiooligosaccharide.

5. The method of any one of claims 1 to 4, wherein the microorganism is yeast, bacteria or mold.

6. The method of any one of claims 1 to 5, wherein the microorganism is a strain selected from the group consisting of *Saccharomyces sp., Bacillus sp., Lactobacillus sp.,* and *Candida sp.*

7. The method of any one of claims 1 to 6, wherein the purification is carried out by decolorization by an activated carbon, demineralization by an ion exchange resin, and evaporation.

8. The method of any one of claims 1 to 7, wherein the high purity gentiooligosaccharide has a purity of at least 90%.

9. High purity gentiooligosaccharide having a purity of at least 90% that is obtained by the method according to any one of claims 1 to 8.

10. A method of improving food flavor or taste by adding the high purity gentiooligosaccharide having a purity of at least 90% according to claim 9 to food.

11. A method of using the high purity gentiooligosaccharide having a purity of at least 90% according to claim 9 as alternatives to food or main ingredient thereof.

12. The method of claim 11, wherein the high purity gentiooligosaccharide having a purity of at least 90% is added in an amount ranging from 0.1 to 40% by weight of the food or main ingredients thereof.

13. The method of claim 11, wherein the food is one or more selected from the group consisting of cocoa, chocolate, coffee, bread or confectionery product, and beverage.
